# EUROPÄISCHE PATENTSCHRIFT

(11) **EP 4 527 300 B1**
(45) Veröffentlichungstag und Bekanntmachung des Hinweises auf die Patenterteilung: **11.02.2026**
(21) Anmeldenummer: 23199109.2
(22) Anmeldetag: 22.09.2023
(51) Int. Cl.: A61B 6/03, A61B 6/00

(54) **COMPUTERTOMOGRAPH MIT VERBESSERTER DATENÜBERTRAGUNG**
COMPUTED TOMOGRAPHY APPARATUS WITH IMPROVED DATA TRANSMISSION
TOMOGRAPHE ASSISTÉ PAR ORDINATEUR AVEC TRANSMISSION DE DONNÉES AMÉLIORÉE

(43) Veröffentlichungstag der Anmeldung: 26.03.2025
(73) Patentinhaber: Siemens Healthineers AG, 91301 Forchheim (DE)
(72) Erfinder: Biele, Markus, 91077 Hetzles (DE); Düppenbecker, Peter Michael, 91074 Herzogenaurach (DE); Hohl, Christoph, 92256 Hahnbach (DE)
(74) Vertreter: Siemens Healthineers Patent Attorneys

(56) Entgegenhaltungen:
- EP-A1- 2 932 901
- US-A- 5 148 448
- US-A1- 2023 000 458

## Beschreibung

Die vorliegende Erfindung geht aus von einem Betriebsverfahren für einen Computertomographen,
- wobei während einer Rotation eines ersten Teils des Computertomographen relativ zu einem zweiten Teil des Computertomographen um eine Rotationsachse zwischen einer Mehrzahl zur von auf dem ersten Teil angeordneten ersten Modems und einer Mehrzahl von auf dem zweiten Teil angeordneten zweiten Modems über eine Mehrzahl von auf dem ersten Teil angeordneten ersten Koppelelementen und eine Mehrzahl von auf dem zweiten Teil angeordneten zweiten Koppelelementen erste Daten übertragen werden,
- wobei die ersten Koppelelemente sich in Umfangsrichtung um die Rotationsachse herum gesehen über einen jeweiligen ersten Winkelbereich erstrecken,
- wobei die ersten Winkelbereiche von erstem Koppelelement zu erstem Koppelelement gesehen zueinander disjunkt sind und über die Gesamtheit der ersten Koppelelemente gesehen einen Vollkreis um die Rotationsachse herum bilden,
- wobei die zweiten Koppelelemente sich um die Rotationsachse herum gesehen über einen jeweiligen zweiten Winkelbereich erstrecken,
- wobei die zweiten Winkelbereiche um die Rotationsachse herum gesehen zueinander disjunkt und voneinander beabstandet sind, insbesondere gleichmäßig um die Rotationsachse herum verteilt angeordnet sind,
- so dass die zweiten Koppelelemente jeweils mit demjenigen ersten Koppelelement koppeln, in dessen erstem Winkelbereich sich der zweite Winkelbereich des jeweiligen zweiten Koppelelements gerade befindet,
- wobei ein Schaltzustand einer zwischen den zweiten Koppelelementen und den zweiten Modems angeordneten Schaltmatrix von einer Einstelleinrichtung dynamisch derart eingestellt wird, dass die ersten Daten stets zwischen denselben ersten und zweiten Modems übertragen werden.

Die vorliegende Erfindung geht weiterhin aus von einem Computertomographen,
- wobei der Computertomograph ein erstes und ein zweites Teil aufweist,
- wobei das erste Teil relativ zum zweiten Teil um eine Rotationsachse rotierbar ist,
- wobei auf dem ersten Teil eine Mehrzahl von ersten Modems angeordnet ist, die mit auf dem ersten Teil angeordneten ersten Koppelelementen datentechnisch verbunden sind, und auf dem zweiten Teil eine Mehrzahl von zweiten Modems angeordnet ist, die mit auf dem zweiten Teil angeordneten zweiten Koppelelementen datentechnisch verbunden sind,
- wobei die ersten Koppelelemente sich in Umfangsrichtung um die Rotationsachse herum gesehen über einen jeweiligen ersten Winkelbereich erstrecken,
- wobei die ersten Winkelbereiche von erstem Koppelelement zu erstem Koppelelement gesehen zueinander disjunkt sind und über die Gesamtheit der ersten Koppelelemente gesehen einen Vollkreis um die Rotationsachse herum bilden,
- wobei die zweiten Koppelelemente sich um die Rotationsachse herum gesehen über einen jeweiligen zweiten Winkelbereich erstrecken,
- wobei die zweiten Winkelbereiche um die Rotationsachse herum gesehen zueinander disjunkt und voneinander beabstandet sind, insbesondere gleichmäßig um die Rotationsachse herum verteilt angeordnet sind,
- so dass die zweiten Koppelelemente jeweils mit demjenigen ersten Koppelelement koppeln, in dessen erstem Winkelbereich sich der zweite Winkelbereich des jeweiligen zweiten Koppelelements gerade befindet,
- wobei zwischen den zweiten Koppelelementen und den zweiten Modems eine Schaltmatrix angeordnet ist und
- wobei der Schaltmatrix eine Einstelleinrichtung zugeordnet ist, von der ein Schaltzustand der Schaltmatrix dynamisch derart eingestellt wird, dass während einer während der Rotation des ersten Teils relativ zum zweiten Teil um die Rotationsachse erfolgenden Datenübertragung erster Daten zwischen den ersten und den zweiten Modems die ersten Daten stets zwischen denselben ersten und zweiten Modems übertragen werden.

Die Erfindung ist in den beigefügten Ansprüchen dargelegt. Computertomographen sind allgemein bekannt.

Bei einem Computertomographen sind auf der Gantry (rotierendes Teil) eine Röntgenquelle und ein Röntgendetektor angeordnet, wobei die Röntgenquelle während des Rotierens der Gantry Röntgenstrahlung emittiert und der Röntgendetektor die emittierte Röntgenstrahlung erfasst. Anhand der so erfassten Bilder eines Untersuchungsobjekts (meist eines Menschen, insbesondere eines Patienten) wird ein dreidimensionales Bild des Untersuchungsobjekts rekonstruiert.

Die Anmelderin weist darauf hin, dass unabhängig vom grammatikalischen Geschlecht eines bestimmten personenbezogenen Begriffs (wie hier beispielsweise des Begriffs "Patient") stets Personen mit männlicher, weiblicher und anderer Geschlechteridentität mit umfasst sind.

Das Rekonstruieren des dreidimensionalen Bildes erfolgt mittels einer Auswertungseinrichtung, die außerhalb der Gantry angeordnet ist, beispielsweise sogar außerhalb des Untersuchungsraums, in dem der Computertomograph angeordnet ist. Die mittels des Röntgendetektors erfassten Bilddaten müssen während des Rotierens von der Gantry zu der Auswertungseinrichtung übertragen werden. In der Regel erfolgt dies über einen Übertragungskanal, über den die digitalen Daten von einer Datenquelle (beispielsweise einer die erfassten Daten des Röntgendetektors geringfügig aufarbeitenden Vorauswertungseinrichtung) einem Modem zugeführt werden, das die digitalen Daten auf ein Trägersignal aufmoduliert. Das modulierte Trägersignal wird über einen Übertragungskanal einem weiteren Modem zugeführt. Das weitere Modem ist am Grundkörper (feststehendes Teil) angeordnet. Es demoduliert das modulierte Trägersignal und führt das so ermittelte Empfangssignal einer Datensinke zu. Die Datenquelle und das zugehörige Modem sind in diesem Fall auf der Gantry angeordnet, das weitere Modem und die Datensinke auf dem feststehenden Grundkörper. Der Übertragungskanal bildet somit die Brücke zwischen der Gantry und dem feststehenden Grundkörper.

Mit der stetigen Weiterentwicklung von Computertomographen steigt das Datenvolumen, das von der Datenquelle zu der Datensinke übertragen werden soll, immer weiter an. Konventionelle Arten der Datenübertragung stoßen zunehmend an Grenzen. Es wird daher versucht, die Datenrate, mit der die Daten übertragen werden, zu erhöhen.

Ein Betriebsverfahren für einen Computertomographen der eingangs genannten Art und der zugehörige Computertomograph sind aus der EP 2 932 901 A1 bekannt.

Aus der US 5 148 448 A ist ein Vorverzerrungsschaltkreis bekannt, der insbesondere in digitalen Datenübertragungssystemen verwendet werden kann, in denen die Daten übertragen werden. Die Daten können unter Verwendung einer Quadraturamplitudenmodulation übertragen werden. Der Vorverzerrungsschaltkreis umfasst eine Rückkopplung, mittels derer der Vorverzerrungsschaltkreis sich nach und nach adaptiert.

Die Aufgabe der vorliegenden Erfindung besteht darin, Möglichkeiten zu schaffen, mittels derer für eine vorgegebene Bandbreite eines Übertragungskanals die Datenrate bei der Datenübertragung zwischen der Gantry und dem feststehenden Grundkörper erhöht werden kann.

Die Aufgabe wird durch ein Betriebsverfahren mit den Merkmalen des Anspruchs 1 gelöst. Vorteilhafte Ausgestaltungen des Betriebsverfahrens sind Gegenstand der abhängigen Ansprüche 2 bis **6.**

Erfindungsgemäß wird ein Betriebsverfahren der eingangs genannten Art dadurch ausgestaltet, dass ein Schaltzustand einer zwischen den zweiten Koppelelementen und den zweiten Modems angeordneten Schaltmatrix von einer Einstelleinrichtung dynamisch derart eingestellt wird, dass die ersten Daten stets zwischen denselben ersten und zweiten Modems übertragen werden.

Das erste Teil des Computertomographen kann ein feststehender Grundkörper des Computertomographen sein. In diesem Fall ist das zweite Teil die rotierende Gantry des Computertomographen. Alternativ kann das erste Teil die rotierende Gantry des Computertomographen sein. In diesem Fall ist das zweite Teil der feststehende Grundkörper. Aufgrund des Umstands, dass beide Zuordnungen möglich sind, kann auch die Datenübertragung nach Bedarf von der Gantry zum Grundkörper oder umgekehrt erfolgen. Die Datenübertragung von der Gantry zum Grundkörper stellt zwar den Regelfall dar, aber auch in die umgekehrte Übertragungsrichtung sind Übertragungen erforderlich.

Im Rahmen der vorliegenden Erfindung stimmt die Anzahl an zweiten Modems mit der Anzahl an ersten Modems überein. Die ersten und zweiten Modems als solche können als sogenannte Backbone-Modems ausgebildet sein, wie sie für Kommunikationssysteme bekannt sind, beispielsweise für die Kommunikation zwischen Mobilfunkmasten. Derartige Modems sind für variierende Signalpfade optimiert und weisen eine interne digitale Signalverarbeitung auf, welche aus FIR-Filtern, Equalizern und einer FEC (forward error correction) besteht. Ein Beispiel eines derartigen Modems ist ein Chip, der von der Firma MaxLinear unter der Typenbezeichnung BCM85110 vertrieben wird.

Die Anzahl an ersten Koppelelementen stimmt in der Regel ebenfalls mit der Anzahl an ersten Modems überein. Die Anzahl an zweiten Koppelelementen ist hingegen in der Regel größer als die Anzahl an ersten Modems. Im optimalen Fall ist sie exakt um 1 größer.

Die ersten Winkelbereiche sind in der Regel untereinander gleich groß. Wenn mit n die Anzahl an ersten Koppelelementen bezeichnet wird, erstrecken sich die ersten Winkelbereiche also jeweils über 360°/n.

Die Erstreckung der zweiten Winkelbereiche kann hingegen erheblich kleiner sein. Wenn mit m die Anzahl an zweiten Koppelelementen bezeichnet wird, erstrecken sich die zweiten Winkelbereiche vorzugsweise jedoch maximal über 360°/nm. Aufgrund der vorzugsweise gleichmäßigen Verteilung sind die zweiten Koppelelemente vorzugsweise um 360°/m voneinander beabstandet. Wenn - beispielsweise n - den Wert 3 aufweist und m den Wert 4 aufweist, sollten sich die zweiten Winkelbereiche also vorzugsweise maximal über 360°/12 = 30° erstrecken und um 360°/4 = 90° voneinander beabstandet sein.

Die Schaltmatrix muss eine Zuordnung der m zweiten Koppelelementen zu den n zweiten Modems vornehmen. Eine geeignete Schaltmatrix kann durch eine parallele Anordnung von n Schaltern ausgebildet sein, die jeweils m Eingänge auf einen (1) Ausgang durchschalten können. Der jeweilige Ausgang kann mit einem der n zweiten Modems fest verbunden sein. Entsprechende Schalter sind bekannt. Rein beispielhaft kann auf den Schalter verwiesen werden, der von der Firma MACOM unter der Typenbezeichnung MASW-011152 vertrieben wird.

Durch das immer wieder erfolgende Umschalten der Schaltmatrix können die Sprünge im Übertragungsverhalten von den ersten zu den zweiten Modems deutlich reduziert werden. Dadurch kann die Rekonstruktion der ersten Daten in qualitativ hochwertiger Art und Weise erfolgen. Dadurch kann die Qualität der Datenübertragung auch während des Rotierens des ersten Teils relativ zum zweiten Teil aufrechterhalten werden.

Vorzugsweise ermittelt die Einstelleinrichtung den Schaltzustand der Schaltmatrix in Abhängigkeit von einem aktuellen Drehwinkel des ersten Teils relativ zum zweiten Teil. Der Einstelleinrichtung ist also der aktuelle Drehwinkel als solcher bekannt und wird von der Einstelleinrichtung zum Ermitteln des Schaltzustands der Schaltmatrix verwendet. Diese Vorgehensweise ist besonders einfach.

Im einfachsten Fall erfolgt das dynamische Einstellen der Schaltmatrix durch die Einstelleinrichtung unter Verwertung ausschließlich des Drehwinkels des ersten Teils relativ zum zweiten Teil. Gegebenenfalls kann die Einstelleinrichtung im Rahmen der Ermittlung des Schaltzustands der Schaltmatrix zusätzlich zu dem Drehwinkel aber auch mindestens eine zeitliche Ableitung des Drehwinkels (also die Drehgeschwindigkeit, die Drehbeschleunigung usw.) berücksichtigen.

Es ist möglich, dass der Drehwinkel jeweils gemessen wird, dass also der Ermittlung der Einstellung der Schaltmatrix jeweils ein gemessener Drehwinkel zu Grunde liegt. Auch in diesem Fall ist es möglich, im Rahmen der Ermittlung der Einstellung der Schaltmatrix zusätzlich auch mindestens eine zeitliche Ableitung des Drehwinkels mit zu berücksichtigen.

Alternativ ist es möglich, dass der Drehwinkel nur bei vorbestimmten Winkelpositionen gemessen wird, beispielsweise nur alle 120°, alle 180° oder sogar nur einmal pro vollständiger Umdrehung des ersten Teils relativ zum zweiten Teil. In diesem Fall wird der Drehwinkel zwischen den vorbestimmten Winkelpositionen von der Einstelleinrichtung durch Fortschreibung des zuletzt gemessenen Drehwinkels anhand von die Drehung charakterisierenden Betriebsdaten ermittelt, insbesondere der Drehzahl und/oder der Drehbeschleunigung.

Vorzugsweise werden die zu übertragenden Daten den ersten oder zweiten Modems als komplexe Signale zugeführt und führen die ersten oder zweiten Modems eine Quadraturamplitudenmodulation eines Trägersignals gemäß den ihnen zugeführten komplexen Signalen durch. Dadurch kann die Datenrate, mit der die ersten Daten übertragen werden können, maximiert werden. Insbesondere in Verbindung mit einer Quadraturamplitudenmodulation zeigt die vorliegende Erfindung ihre vollen Vorteile.

Das dynamische Einstellen der Verzerrungsvorschriften durch die Einstelleinrichtung kann ebenfalls in direkter Abhängigkeit vom Drehwinkel des ersten Teils relativ zum zweiten Teil vorgenommen werden. Gegebenenfalls kann wieder zusätzlich zum Drehwinkel auch mindestens eine zeitliche Ableitung des Drehwinkels mit verwertet werden.

Durch derartige Verzerrungen kann die durch die Übertragungsfunktion des jeweiligen Übertragungskanals bewirkte Verzerrung einer Datenübertragung kompensiert werden. Somit kann ein im Ergebnis nahezu unverzerrtes Signal zurückgewonnen werden, so dass eine qualitativ hochwertige Rekonstruktion der ersten Daten möglich ist. Es erfolgt also ein Angepasstes Filtern (Matched Filtering). Eine derartige Vorgehensweise ist im Stand der Technik bei einer Quadraturamplitudenmodulation vom Ansatz her bekannt. Vorliegend kommt jedoch hinzu, dass die Verzerrungsvorschriften in Abhängigkeit vom Drehwinkel des ersten Teils relativ zum zweiten Teil mehr oder minder kontinuierlich nachgeführt werden. Dadurch kann die hohe Qualität der Datenübertragung auch während des Rotierens der Gantry aufrechterhalten werden.

Die erforderlichen Parameter zum Einstellen der Verzerrungseinrichtungen können beispielsweise in der Einstelleinrichtung in einer Lookup-Table für eine Vielzahl von Eingangsgrößen hinterlegt sein. Die Eingangsgrößen umfassen in der Regel zumindest den Drehwinkel des ersten Teils relativ zum zweiten Teil, gegebenenfalls auch zeitliche Ableitungen des Drehwinkels.

Vorzugsweise werden während der Rotation des ersten Teils relativ zum zweiten Teil über die ersten und zweiten Koppelelemente zusätzlich zwischen auf dem ersten Teil angeordneten dritten Modems und auf dem zweiten Teil angeordneten vierten Modems zweite Daten übertragen. Die Schaltmatrix wird in diesem Fall von der Einstelleinrichtung dynamisch derart geschaltet, dass die zweiten Daten stets zwischen denselben dritten und vierten Modems übertragen werden. Dadurch kann die Übertragungsrate noch weiter erhöht werden.

Vorzugsweise werden die ersten und die zweiten Daten mittels der ersten bis vierten Modems vor dem Zuführen zu den ersten und zweiten Koppelelementen auf Trägersignale verschiedener Frequenzen aufmoduliert, so dass die jeweils belegten Frequenzbereiche zueinander disjunkt sind.

Die Aufgabe wird weiterhin durch einen Computertomographen mit den Merkmalen des Anspruchs 7 gelöst. Vorteilhafte Ausgestaltungen des Computertomographen sind Gegenstand der abhängigen Ansprüche 8 bis 12.

Erfindungsgemäß wird ein Computertomograph der eingangs genannten Art dadurch ausgestaltet, dass zwischen den ersten Modems und den ersten Koppelelementen und/oder zwischen den zweiten Modems und den zweiten Koppelelementen Verzerrungseinrichtungen angeordnet sind, mittels derer eine Verzerrung des jeweils übertragenen Signals gemäß einer jeweiligen Verzerrungsvorschrift erfolgt, und dass die jeweilige Verzerrungsvorschrift von der Einstelleinrichtung in Abhängigkeit vom Drehwinkel des ersten Teils relativ zum zweiten Teil eingestellt wird.

Die dadurch bewirkten Sachverhalte und Vorteile korrespondieren mit denen des erfindungsgemäßen Betriebsverfahrens.

Die vorteilhaften Ausgestaltungen des Computertomographen korrespondieren mit den vorteilhaften Ausgestaltungen des Betriebsverfahrens. Gleiches gilt für die dadurch bewirkten Vorteile.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die in Verbindung mit den Zeichnungen näher erläutert werden. Hierbei zeigen in schematischer Darstellung:
- FIG 1: einen Computertomographen,
- FIG 2: ein Zeitdiagramm,
- FIG 3: ein weiteres Zeitdiagramm,
- FIG 4: eine Datenübertragungsstruktur,
- FIG 5: ein Winkeldiagramm,
- FIG 6: ein Winkeldiagramm,
- FIG 7: ein Winkeldiagramm,
- FIG 8: eine Datenquelle und ein erstes Modem,
- FIG 9: ein zweites Modem und eine Datensinke,
- FIG 10: einen Kommunikationsweg,
- FIG 11: eine weitere Datenübertragungsstruktur,
- FIG 12: ein Frequenzdiagramm und
- FIG 13: ein weiteres Frequenzdiagramm.

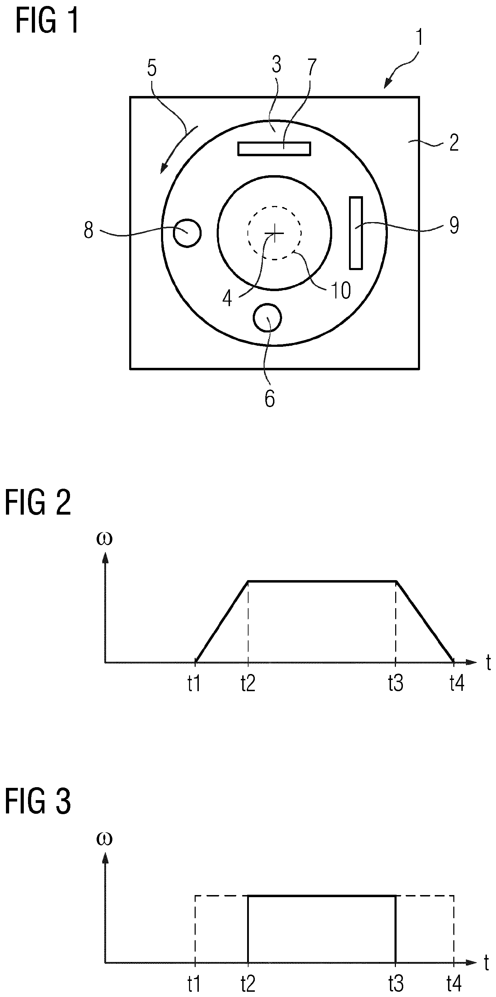

Gemäß FIG 1 weist ein Computertomograph 1 einen feststehenden Grundkörper 2 auf. An dem Grundkörper 2 ist eine Gantry 3 drehbar gelagert, so dass die Gantry 3 relativ zum Grundkörper 2 um eine Rotationsachse 4 rotierbar ist. Die Rotierbarkeit ist in FIG 1 durch einen Pfeil 5 angedeutet. Die Gantry 3 trägt, wie allgemein üblich, eine Röntgenquelle 6 und einen Röntgendetektor 7. Die Röntgenquelle 6 und der Röntgendetektor 7 liegen einander bezüglich der Rotationsachse 4 diametral gegenüber. In manchen Fällen trägt die Gantry 3 zusätzlich auch eine weitere Röntgenquelle 8 und einen weiteren Röntgendetektor 9. Auch die weitere Röntgenquelle 8 und der weitere Röntgendetektor 9 liegen einander bezüglich der Rotationsachse 4 diametral gegenüber. Die weitere Röntgenquelle 8 und der weitere Röntgendetektor 9 sind, sofern sie vorhanden sind, in aller Regel bezüglich der erstgenannten Röntgenquelle 6 und dem erstgenannten Röntgendetektor 7 um 90° in Umfangsrichtung um die Rotationsachse 4 herum gesehen versetzt angeordnet. Während des Rotierens der Gantry 3 um die Rotationsachse 4 können mittels des Röntgendetektors 7 bzw. der Röntgendetektoren 7, 9 Röntgenbilder eines Untersuchungsobjekts 10 erfasst werden, das im Bereich der Rotationsachse 4 angeordnet ist.

Der Grundkörper 2 kann als erstes Teil im Sinne der vorliegenden Erfindung angesehen werden. In diesem Fall ist die Gantry 3 ein zweites Teil im Sinne der vorliegenden Erfindung. Es ist aber auch die umgekehrte Zuordnung möglich. In Verbindung mit der vorstehend erläuterten Ausgestaltung wird nachstehend eine Datenübertragung von der Gantry 3 zum Grundkörper 2 erläutert. Prinzipiell ist aber auch eine Datenübertragung vom Grundkörper 2 zur Gantry 3 möglich. Entscheidend ist immer nur die relative Bewegbarkeit der beiden Teile 2, 3 zueinander und die Übertragung von Daten von dem einen der beiden Teile 2, 3 zum anderen der beiden Teile 2, 3.

FIG 2 zeigt das Rotieren der Gantry 3 als Funktion der Zeit t. Gemäß FIG 2 wird zum Erfassen der Röntgenbilder des Untersuchungsobjekts zu einem Zeitpunkt t1 mit dem Rotieren der Gantry 3 begonnen. Konkret wird die Gantry 3 ab dem Zeitpunkt t1 bis zu einem Zeitpunkt t2 beschleunigt, so dass eine Drehgeschwindigkeit ω zum Zeitpunkt t2 einen Maximalwert erreicht. Danach rotiert die Gantry 3 bis zu einem Zeitpunkt t3 mit der maximalen Drehgeschwindigkeit und wird schließlich wieder abgebremst, bis sie zu einem Zeitpunkt t4 zum Stillstand kommt.

Das Erfassen der Röntgenbilder erfolgt zumindest zwischen den Zeitpunkten t2 und t3, oftmals sogar ab dem Zeitpunkt t1 und bis zum Zeitpunkt t4. Mehr oder minder simultan zum Erfassen der Röntgenbilder werden die Röntgenbilder gemäß FIG 3 auch von der Gantry 3 an den Grundkörper 2 übertragen. FIG 3 zeigt in einer durchgezogenen Linie den Zeitraum, während dessen zumindest die Datenübertragung erfolgt, und in gestrichelten Linien die Zeiträume, während derer ebenfalls eine Datenübertragung erfolgen kann. Die übertragenen Daten sind digitaler Natur.

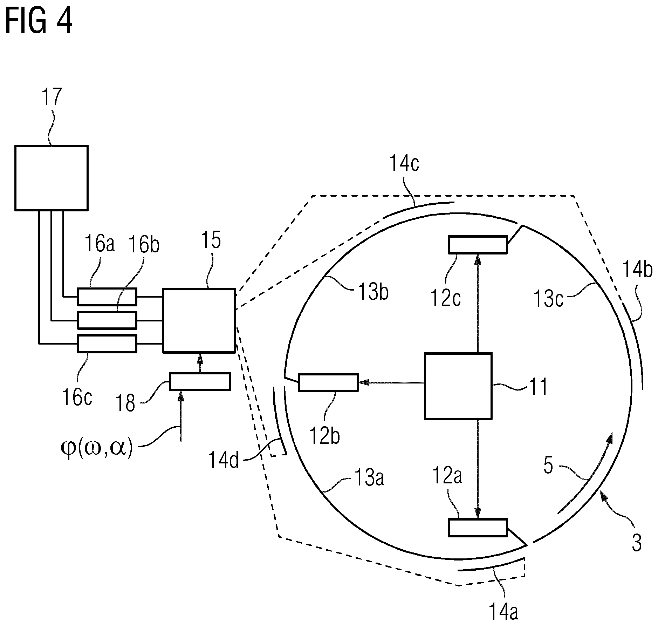

FIG 4 zeigt in vereinfachter Form eine Struktur zur Übertragung der Daten zwischen der Gantry 3 und dem Grundkörper 2.

Gemäß FIG 4 ist eine Datenquelle 11 vorhanden. Die Datenquelle 11 kann beispielsweise eine Einheit sein, welche von den Röntgendetektoren 7, 9 deren Bilder entgegennimmt und sie (geringfügig) aufbereitet. Die Datenquelle 11 ist mit einer Mehrzahl von ersten Modems 12 datentechnisch verbunden. Vorliegend sind drei erste Modems 12 vorhanden. Die ersten Modems 12 sind mit einem jeweiligen ersten Koppelelement 13 datentechnisch verbunden. Die ersten Koppelelemente 13 erstrecken sich jeweils über 360°/n um die Rotationsachse 4 herum, wobei n die Anzahl an ersten Modems 12 bzw. ersten Koppelelementen 13 ist. Der Winkelbereich, über den sich ein jeweiliges erstes Koppelelement 13 erstreckt, ist disjunkt zu den Winkelbereichen, über die sich die anderen ersten Koppelelementen 13 erstrecken. Zusammen bilden die genannten Winkelbereiche einen Vollkreis. Die ersten Koppelelemente 13 können beispielsweise als dielektrische Leiter oder als geschlitzte Wellenleiter ausgebildet sein. Die Datenquelle 11, die ersten Modems 12 und die ersten Koppelelemente 13 sind auf der Gantry 3 angeordnet.

Die ersten Koppelelemente 13 koppeln mit zweiten Koppelelementen 14. Die Anzahl an zweiten Koppelelementen 14 ist meist größer als die Anzahl an ersten Koppelelementen 13. Vorliegend liegt die Anzahl an zweiten Koppelelementen 14 bei 4, ist also um 1 größer als die Anzahl an ersten Koppelelementen 13. Die zweiten Koppelelemente 14 erstrecken sich um die Rotationsachse 4 herum gesehen ebenfalls über einen jeweiligen Winkelbereich. Diese Winkelbereiche sind jedoch erheblich kleiner als die Winkelbereiche, über welche sich die ersten Koppelelemente 13 erstrecken. Im Idealfall sind die Winkelbereiche sehr klein (nur wenige Grad). Die Winkelbereiche, über welche sich die zweiten Koppelelemente 14 erstrecken, sind in jedem Fall disjunkt und voneinander beabstandet. Vorzugsweise sind die Winkelbereiche gleichmäßig um die Rotationsachse 4 herum verteilt angeordnet.

Die zweiten Koppelelemente 14 sind über eine Schaltmatrix 15 mit zweiten Modems 16 datentechnisch verbunden. Die Schaltmatrix 15 ist also zwischen den zweiten Koppelelementen 14 und den zweiten Modems 16 angeordnet. Die zweiten Modems 16 wiederum sind mit einer Datensinke 17 datentechnisch verbunden. Die zweiten Koppelelemente 14 und die zweiten Modems 16 sind - ebenso wie die Schaltmatrix 15 - auf dem Grundkörper 2 angeordnet.

Die ersten Modems 12, die ersten Koppelelemente 13 und die zweiten Modems 16 sind in FIG 4 jeweils mit einem Zusatz a bis c versehen, um sie im Rahmen späterer Erläuterungen bei Bedarf voneinander unterscheiden zu können. Aus den gleichen Gründen sind in FIG 4 die zweiten Koppelelemente 14 jeweils mit einem Zusatz a bis d versehen.

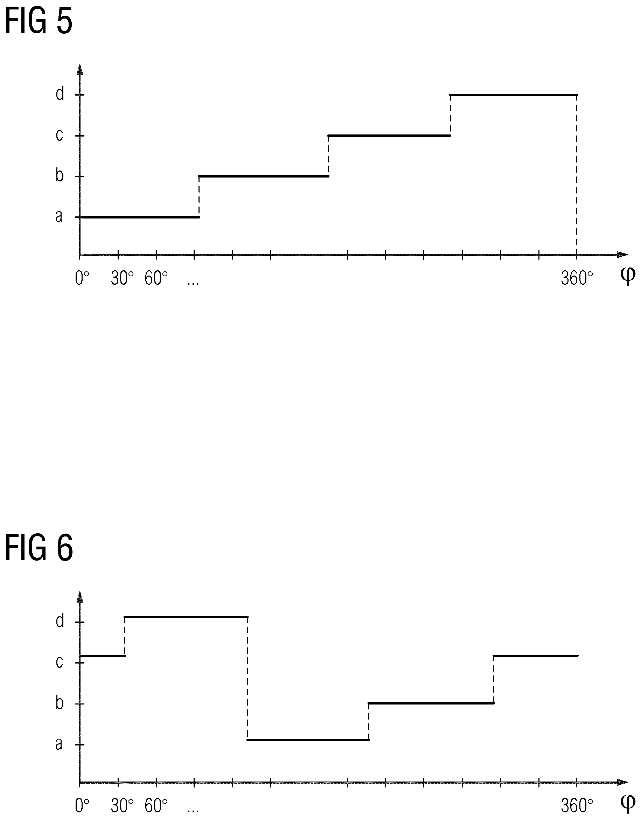

Aus den vorstehenden Erläuterungen ist ersichtlich, dass die Übertragung der Daten zwischen den ersten Modems 12 und den zweiten Modems 16 über die ersten Koppelelemente 13 und die zweiten Koppelelemente 14 erfolgt. Weiterhin ist ersichtlich, dass eine Kopplung zwischen einem der ersten Koppelelemente 13 und einem der zweiten Koppelelemente 14 nur dann besteht, wenn sich der Winkelbereich, den das jeweilige zweite Koppelelement 14 überdeckt, in dem Winkelbereich befindet, den das jeweilige erste Koppelelement 13 überdeckt. Die Kopplung der ersten und der zweiten Koppelelemente 13, 14 miteinander variiert also beim Rotieren der Gantry **3.** Dies wird nachstehend in Verbindung mit den FIG 5 bis 7 näher erläutert. Im Rahmen dieser Erläuterung wird angenommen, dass die in FIG 4 dargestellte Konfiguration gegeben ist, dass also insgesamt drei erste Koppelelemente 13 vorhanden sind, die ersten Koppelelemente 13 sich über je 120° erstrecken, insgesamt vier zweite Koppelelemente 14 vorhanden sind, die zweiten Koppelelemente 14 um jeweils 90° gegeneinander versetzt angeordnet sind und die zweiten Koppelelemente 14 sich über jeweils 30° erstrecken.

In den FIG 5 bis 7 ist auf der Abszisse jeweils der Drehwinkel φ von 0° bis 360° eingetragen. Ein Drehwinkel φ von 0° kann beispielsweise der in FIG 4 dargestellten Drehstellung entsprechen. Eine Drehung entgegen dem Uhrzeigersinn soll mit einem Ansteigen des Drehwinkels φ korrespondieren. Auf der Ordinate ist eingetragen, mit welchem der zweiten Koppelelemente 14 das jeweilige erste Koppelelement 13 koppelt. Die FIG 5 bis 7 zeigen - in dieser Reihenfolge - die Kopplung für das erste Koppelelement 13a, das erste Koppelelement 13b und das erste Koppelelement 13c.

Ein Schaltzustand der Schaltmatrix 15 orientiert sich an dem vorstehend in Verbindung mit den FIG 5 bis 7 geschilderten Sachverhalt: Der Schaltzustand wird von einer der Schaltmatrix 15 zugeordneten Einstelleinrichtung 18 (siehe FIG 4) dynamisch derart eingestellt, dass die Daten stets zwischen denselben ersten und zweiten Modems 12, 16 übertragen werden. Das zweite Modem 16a wird also stets mit demjenigen zweiten Koppelelement 14 verbunden, das gerade über das erste Koppelelement 13a von dem ersten Modem 12a Daten erhält. In analoger Weise wird das zweite Modem 16b stets mit demjenigen zweiten Koppelelement 14 verbunden, das gerade über das erste Koppelelement 13b von dem ersten Modem 12b Daten erhält. Und auch das zweite Modem 16c wird stets mit demjenigen zweiten Koppelelement 14 verbunden, das gerade über das erste Koppelelement 13c von dem ersten Modem 12c Daten erhält. Gleiches würde im Übrigen auch bei einer Kommunikation in die umgekehrte Richtung gelten.

Um den Schaltzustand der Schaltmatrix 15 bestimmen zu können und demzufolge die Schaltmatrix 15 entsprechend einstellen zu können, muss der Einstelleinrichtung 18 direkt oder indirekt der Drehwinkel φ der Gantry 3 bekannt sein. Im einfachsten Fall wird der Drehwinkel φ der Einstelleinrichtung 18 direkt zugeführt. Gegebenenfalls können der Einstelleinrichtung 18 - sei es zusätzlich, sei es alternativ - auch die Drehgeschwindigkeit ω und/oder die Drehbeschleunigung α zugeführt werden. In diesem Fall kann die Einstelleinrichtung 18 diese Werte ω, α bei der Ermittlung mit berücksichtigen oder (beispielsweise in Verbindung mit einem Referenzpuls, der einmal pro Umdrehung bei Passieren einer Referenzdrehstellung abgegeben wird) den Drehwinkel φ ermitteln. Durch zusätzliche Berücksichtigung der Drehgeschwindigkeit ω und/oder der Drehbeschleunigung α können eventuelle zeitliche Versätze besser berücksichtigt werden.

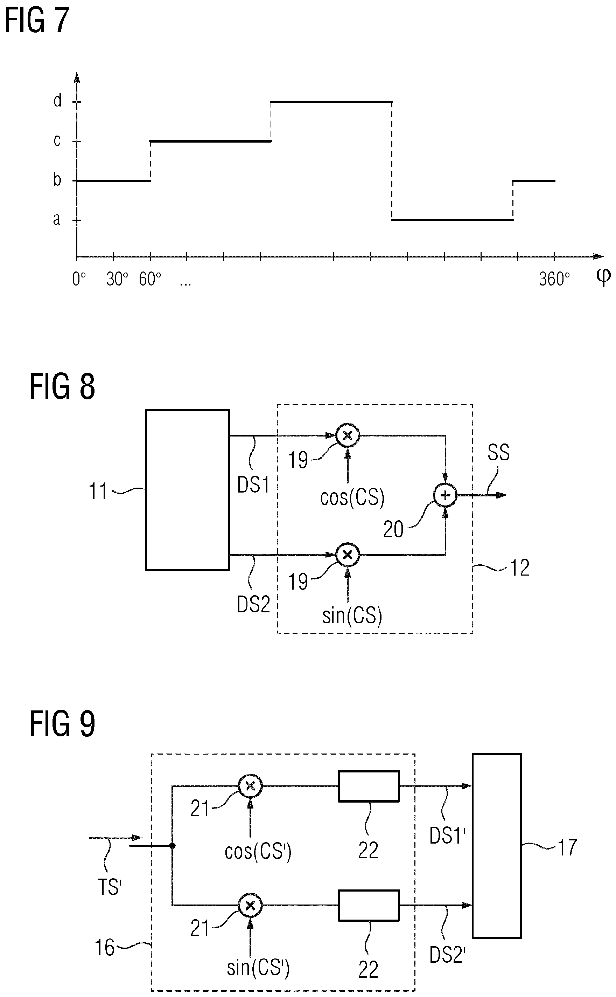

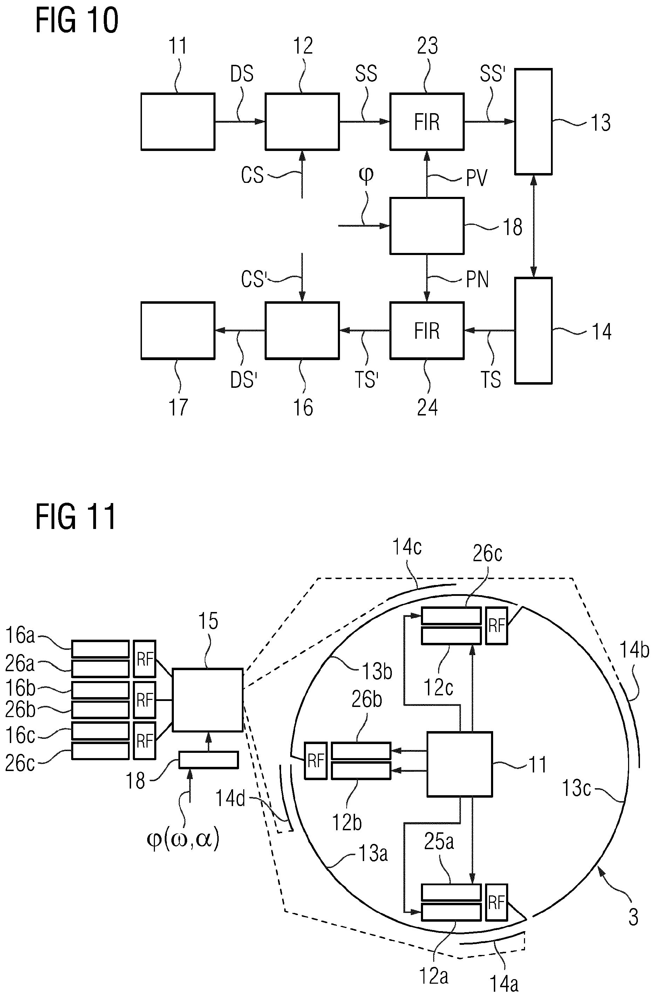

Die Datenquelle 11 führt entsprechend der Darstellung in FIG 8 einen Datenstrom DS (also die zu übertragenden Daten, siehe FIG 10) dem jeweiligen ersten Modem 12 vorzugsweise als komplexes Signal zu. Der Datenstrom DS umfasst somit zwei Teilsignale DS1, DS2, die den Realteil und den Imaginärteil des komplexen Signals darstellen. Das jeweilige erste Modem 12 ist in diesem Fall als Quadraturamplitudenmodulator ausgebildet. Es umfasst zwei Multiplizierer 19, denen der Kosinus und der Sinus eines Trägersignals CS zugeführt werden. Den beiden Multiplizierern 19 wird weiterhin jeweils eines der beiden Teilsignale DS1, DS2 zugeführt. Die von den beiden Multiplizierern 19 generierten Signale werden einem Addierer 20 zugeführt, der die beiden Signale zu einem Sendesignal SS addiert. Das Sendesignal SS wird über das jeweilige erste Koppelelement 13 und das jeweilige zweite Koppelelement 14 zum entsprechenden zweiten Modem 16 übertragen.

Im Falle einer Quadraturamplitudenmodulation sind gemäß der Darstellung in FIG 9 auch die zweiten Modems 16 und die Datensinke 17 entsprechend ausgebildet. Insbesondere umfasst in diesem Fall das jeweilige zweite Modem 16 zwei Multiplizierer 21, denen zum einen das übertragene Signal DS' (siehe FIG 10) und der Kosinus und der Sinus eines mit dem Trägersignal CS frequenzgleichen weiteren Trägersignals CS' zugeführt werden. Die beiden Multiplizierer 21 liefern als Ausgangssignale den Realteil DS1' und den Imaginärteil DS2' des demodulierten Signals, denen ein jeweiliger hochfrequenter Anteil überlagert ist. Der jeweilige hochfrequente Anteil wird in einem jeweiligen Tiefpassfilter 22 ausgefiltert, so dass am Ausgang des jeweiligen Tiefpassfilters 22 der Realteil DS1' und der Imaginärteil DS2' des demodulierten (komplexen) Signals zur Verfügung stehen. Die beiden Teilsignale DS1', DS2' werden der Datensinke 17 zugeführt.

Gemäß FIG 10 werden die Daten in Form eines Datenstroms DS von der Datenquelle 11 über eines der ersten Modems 12, das zugehörige erste Koppelelement 13 und eines der zweiten Koppelelemente 14 zum entsprechenden zweiten Modem 16 zur Datensinke 17 übertragen. Die Schaltmatrix 15 ist in FIG 10 nicht mit dargestellt. Sie ist jedoch vorhanden.

Zur korrekten Demodulation muss die Phase des weiteren Trägersignals CS' mit der Phase des Trägersignals CS übereinstimmen. Zu diesem Zweck können entsprechend der Darstellung in FIG 10 zwischen den ersten Modems 12 und den ersten Koppelelementen 13 erste Verzerrungseinrichtungen 23 angeordnet. Alternativ oder zusätzlich können zwischen den zweiten Koppelelementen 14 und den zweiten Modems 16 zweite Verzerrungseinrichtungen 24 angeordnet sein. Die Verzerrungseinrichtungen 23, 24 können insbesondere als FIR-Filter ausgebildet sein. Die genaue Abfolge bei der Datenübertragung ist somit wie folgt:
Die Datenquelle 11 übermittelt an das entsprechende erste Modem 12 den Datenstrom DS. Der Datenstrom DS kann, siehe die Ausführungen zu FIG 8, insbesondere als komplexes Signal ausgebildet sein. Das erste Modem 12 moduliert das Trägersignal CS gemäß dem Datenstrom DS. Das modulierte Trägersignal entspricht dem Sendesignal SS, das das erste Modem 12 vom Ansatz her in das erste Koppelelement 13 einspeisen soll. Das Sendesignal SS wird jedoch zunächst der ersten Verzerrungseinrichtung 23 zugeführt, welche das Sendesignal SS gemäß einer ersten Verzerrungsvorschrift verzerrt. Erst das verzerrte Sendesignal - nachfolgend zur Unterscheidung von dem ursprünglichen Sendesignal SS mit dem Bezugszeichen SS' versehen - wird dem entsprechenden ersten Koppelelement 13 zugeführt.

In analoger Weise wird das übertragene Signal TS zunächst der zweiten Verzerrungseinrichtung 24 zugeführt. Die zweite Verzerrungseinrichtung 24 verzerrt das übertragene Signal TS gemäß einer zweiten Verzerrungsvorschrift. Erst das verzerrte übertragene Signal - nachfolgend zur Unterscheidung von dem ursprünglichen übertragenen Signal TS mit dem Bezugszeichen TS' versehen - wird dem entsprechenden zweiten Modem 16 zugeführt. Das zweite Modem 16 demoduliert das verzerrte übertragene Signal TS' und generiert dadurch einen übertragenen Datenstrom DS'. Der übertragene Datenstrom DS' wird der Datensinke 16 zugeführt.

Die Verzerrungseinrichtungen 23, 24 können mit Parametern PV, PN parametriert werden. Die Parameter PV bestimmen die erste Verzerrungsvorschrift. In analoger Weise bestimmen die Parameter PN bestimmen die zweite Verzerrungsvorschrift.

Wie bereits erwähnt, erfolgen die Datenübertragungen während des Rotierens der Gantry 3. Demzufolge ändert sich während der Datenübertragungen laufend der Bereich des jeweiligen ersten Koppelelements 13, an dem das jeweils angrenzende zweite Koppelelement 14 mit dem jeweiligen ersten Koppelelement 13 koppelt. Demzufolge ändert sich während des Rotierens der Gantry 3 laufend die effektive Länge des Übertragungskanals vom jeweiligen ersten Modem 12 zum jeweiligen zweiten Modem 16. Demzufolge ändern sich auch die Übertragungseigenschaften des Übertragungskanals. Der Sinn und Zweck der Vorgehensweise von FIG 10 besteht darin, durch entsprechende Einstellung der Verzerrungseinrichtungen 23, 24 bzw. entsprechende Vorgabe der Parameter PV, PN die Übertragungseigenschaften der Gesamtheit von erster Verzerrungseinrichtung 23, Übertragungskanal und zweiter Verzerrungseinrichtung 24 konstant bzw. zumindest im Wesentlichen konstant zu halten,

Zum Konstanthalten ist es erforderlich, während des Rotierens der Gantry 3 auch die Parameter PV, PN dynamisch einzustellen. Das Einstellen kann, wie aus FIG 10 ersichtlich ist, insbesondere durch die Einstelleinrichtung 18 erfolgen. Das Einstellen kann insbesondere in Abhängigkeit vom Drehwinkel φ, gegebenenfalls unter zusätzliche Berücksichtigung der Drehgeschwindigkeit ω und/oder der Drehbeschleunigung α erfolgen. Die entsprechenden Ausführungen zum Einstellen der Schaltmatrix 15 sind in analoger Weise anwendbar.

Die Ermittlung der jeweiligen Einstellung der Schaltmatrix 15 ist relativ einfach. Die Ermittlung der Parameter PV, PN kann hingegen erheblich komplexer sein und muss auch viel häufiger vorgenommen werden. Vorzugsweise ist daher in der Einstelleinrichtung 18 eine Lookup-Table hinterlegt, in welcher die Parameter PV, PN für bestimmte Werte des Drehwinkels φ, gegebenenfalls zusätzlich auch für bestimmte Werte der Drehgeschwindigkeit ω und/oder der Drehbeschleunigung α, hinterlegt sind. Die Verwendung einer Lookup-Table gewährleistet auf einfache Weise die Echtzeitfähigkeit.

Nachstehend wird das Grundprinzip zur Bestimmung der Parameter PV, PN erläutert.

Wenn mit H, U und V die Übertragungsfunktionen des Übertragungskanals, der ersten Verzerrungseinrichtung 23 und der zweiten Verzerrungseinrichtung 24 bezeichnet werden, so gilt stets H' = UHV, wobei H' die resultierende Übertragungsfunktion von erster Verzerrungseinrichtung 23, Übertragungskanal und zweiter Verzerrungseinrichtung 24 ist. Idealerweise sollte weiterhin die Beziehung H' = I gelten, wobei I die Einheits-Übertragungsfunktion ist.

Die Übertragungsfunktion H des Übertragungskanals ist aufgrund der Rotation der Gantry 3 zeitlich variabel. Die Parameter PV, PN, mit denen die beiden Verzerrungseinrichtungen 23, 24 parametriert werden und die somit die entsprechenden Verzerrungsvorschriften und damit deren Übertragungsfunktionen U, V festlegen, sollten also jederzeit derart bestimmt werden, dass die genannte Beziehung H' = I exakt oder zumindest näherungsweise gilt.

Je nach Sachlage kann es ausreichend sein, wenn nur entweder die erste Verzerrungseinrichtung 23 vorhanden ist oder die zweite Verzerrungseinrichtung 24 vorhanden ist oder zwar beide Verzerrungseinrichtungen 23, 24 vorhanden sind, aber nur die Parameter PV, PN einer der beiden Verzerrungseinrichtungen 23, 24 immer wieder neu eingestellt werden. In aller Regel ist es jedoch vorzuziehen, wenn sowohl beide Verzerrungseinrichtungen 23, 24 vorhanden sind und auch die Parameter PV, PN, beider Verzerrungseinrichtungen 23, 24 immer wieder neu eingestellt werden.

FIG 11 zeigt eine Modifikation der Datenübertragungsstruktur von FIG 4. Gemäß FIG 11 ist auf der Gantry 3 eine Mehrzahl von dritten Modems 25 angeordnet. Die Anzahl an dritten Modems 25 korrespondiert in aller Regel mit der Anzahl an ersten Modems 12. Die dritten Modems 25 sind mit den ersten Koppelelementen 13 datentechnisch verbunden. In analoger Weise ist auf dem Grundkörper 2 eine Mehrzahl von vierten Modems 26 angeordnet. Die Anzahl an vierten Modems 26 korrespondiert mit der Anzahl an dritten Modems 25. Die vierten Modems 26 sind mit den zweiten Koppelelementen 14 über die Schaltmatrix 15 datentechnisch verbunden. Analog zur Vorgehensweise bei den ersten und zweiten Modems 12, 16 wird die Schaltmatrix 15 von der Einstelleinrichtung 18 während der Rotation der Gantry 3 dynamisch derart geschaltet, dass weitere Daten stets zwischen denselben dritten und vierten Modems 25, 26 übertragen werden. Es erfolgt also stets eine Datenübertragung zwischen dem dritten Modem 25a und dem vierten Modem 26a, zwischen dem dritten Modem 25b und dem vierten Modem 26b und zwischen dem dritten Modem 25c und dem vierten Modem 26c. Somit ist während der Rotation der Gantry 3 über die ersten und zweiten Koppelelemente 13, 14 auf völlig analoge Weise eine weitere zusätzliche Kommunikation realisierbar. Die Kommunikation kann in der gleichen Richtung wie die Datenübertragung zwischen den ersten und zweiten Modems 12, 16 erfolgen, also von den dritten Modems 25 zu den vierten Modems 26. Es ist aber auch möglich, eine Datenübertragung in die andere Richtung zu realisieren, so dass also simultan gegenläufige Datenübertragungen erfolgen.

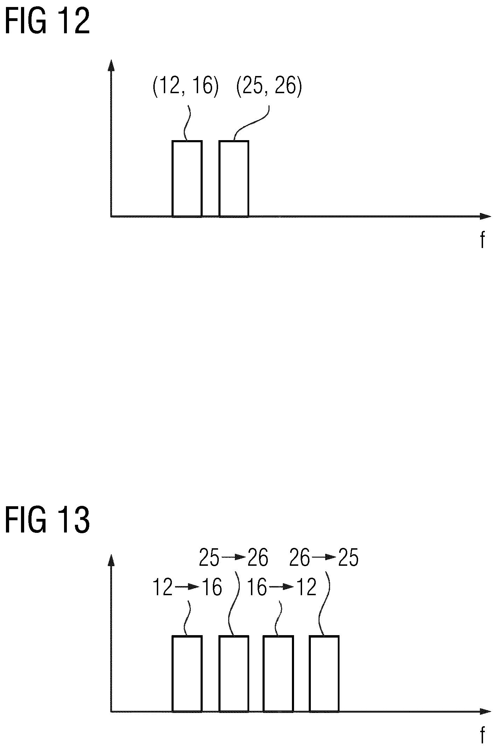

Um die beiden Datenübertragungen über ein jeweiliges erstes Koppelelement 13 zu realisieren, werden die entsprechenden Daten mittels der entsprechenden Modems 12, 25 oder 12, 26 vor dem Zuführen zu den ersten und zweiten Koppelelementen 13, 14 auf Trägersignale verschiedener Frequenzen aufmoduliert, so dass die jeweils belegten Frequenzbereiche zueinander disjunkt sind. FIG 12 zeigt dies beispielhaft für eine Paarung eines der ersten Modems 12 und eines der zweiten Modems 16 einerseits sowie eine Paarung eines der dritten Modems 25 und eines der vierten Modems 26 andererseits. Ersichtlich sind die Frequenzen f, die für die Datenübertragung zwischen dem entsprechenden ersten Modem 12 und dem entsprechenden zweiten Modem 16 genutzt werden, disjunkt von den Frequenzen f, die für die Datenübertragung zwischen dem entsprechenden dritten Modem 25 und dem entsprechenden vierten Modem 26 genutzt werden.

Es ist sogar entsprechend der Darstellung in FIG 13 möglich, über beide Paarungen von Modems 12, 16, 25, 26 simultan in beide Richtungen Daten zu übertragen, sofern die Grundbedingung, dass die jeweils belegten Frequenzbereiche zueinander disjunkt sind, erhalten bleibt.

Zusammengefasst betrifft die vorliegende Erfindung somit folgenden Sachverhalt

Während einer Rotation eines ersten Teils 3 eines Computertomographen 1 relativ zu einem zweiten Teil 2 des Computertomographen 1 um eine Rotationsachse 4 werden zwischen auf dem ersten Teil 3 angeordneten ersten Modems 12 und auf dem zweiten Teil 2 angeordneten zweiten Modems 16 über auf dem ersten Teil 3 angeordnete erste Koppelelemente 13 und auf dem zweiten Teil 2 angeordnete zweite Koppelelemente 14 Daten übertragen. Die ersten Koppelelemente 13 erstrecken sich in Umfangsrichtung um die Rotationsachse 4 herum gesehen über einen jeweiligen ersten Winkelbereich. Diese Winkelbereiche sind von erstem Koppelelement 13 zu erstem Koppelelement 13 gesehen zueinander disjunkt und bilden über die Gesamtheit der ersten Koppelelemente 13 gesehen einen Vollkreis um die Rotationsachse 4 herum. Die zweiten Koppelelemente 14 erstrecken sich um die Rotationsachse 4 herum gesehen über einen jeweiligen zweiten Winkelbereich. Die zweiten Winkelbereiche sind um die Rotationsachse 4 herum gesehen zueinander disjunkt und voneinander beabstandet. Somit koppeln die zweiten Koppelelemente 14 jeweils mit demjenigen ersten Koppelelement 13, in dessen erstem Winkelbereich sich der zweite Winkelbereich des jeweiligen zweiten Koppelelements 14 gerade befindet. Ein Schaltzustand einer zwischen den zweiten Koppelelementen 14 und den zweiten Modems 16 angeordneten Schaltmatrix 15 wird von einer Einstelleinrichtung 18 dynamisch derart eingestellt, dass die ersten Daten stets zwischen denselben ersten und zweiten Modems 12, 16 übertragen werden. Mittels zwischen den ersten Modems 12 und den ersten Koppelelementen 13 und/oder zwischen den zweiten Modems 16 und den zweiten Koppelelementen 14 angeordneter Verzerrungseinrichtungen 23, 24 erfolgt eine Verzerrung des jeweils übertragenen Signals gemäß einer jeweiligen Verzerrungsvorschrift. Die jeweilige Verzerrungsvorschrift wird von der Einstelleinrichtung 18 in Abhängigkeit vom Drehwinkel φ des ersten Teils 3 relativ zum zweiten Teil 2 eingestellt.

Die vorliegende Erfindung weist viele Vorteile auf. Insbesondere wird eine vergleichsweise einfache und dennoch robuste Datenübertragung geschaffen, mittels derer hohe Übertragungsraten im Bereich von etlichen Gigabit pro Sekunde realisiert werden können. Die erfindungsgemäßen Maßnahmen sind auch ohne weiteres geeignet, bei einem bereits bestehenden, noch nicht erfindungsgemäßen Computertomographen nachgerüstet zu werden.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Betriebsverfahren für einen Computertomographen (1),
- wobei während einer Rotation eines ersten Teils (3) des Computertomographen (1) relativ zu einem zweiten Teil (2) des Computertomographen (1) um eine Rotationsachse (4) zwischen einer Mehrzahl von auf dem ersten Teil (3) angeordneten ersten Modems (12) und einer Mehrzahl von auf dem zweiten Teil (2) angeordneten zweiten Modems (16) über eine Mehrzahl von auf dem ersten Teil (3) angeordneten ersten Koppelelementen (13) und eine Mehrzahl von auf dem zweiten Teil (2) angeordneten zweiten Koppelelementen (14) erste Daten übertragen werden,
- wobei die ersten Koppelelemente (13) sich in Umfangsrichtung um die Rotationsachse (4) herum gesehen über einen jeweiligen ersten Winkelbereich erstrecken,
- wobei die ersten Winkelbereiche von erstem Koppelelement (13) zu erstem Koppelelement (13) gesehen zueinander disjunkt sind und über die Gesamtheit der ersten Koppelelemente (13) gesehen einen Vollkreis um die Rotationsachse (4) herum bilden,
- wobei die zweiten Koppelelemente (14) sich um die Rotationsachse (4) herum gesehen über einen jeweiligen zweiten Winkelbereich erstrecken,
- wobei die zweiten Winkelbereiche um die Rotationsachse (4) herum gesehen zueinander disjunkt und voneinander beabstandet sind, insbesondere gleichmäßig um die Rotationsachse (4) herum verteilt angeordnet sind,
- so dass die zweiten Koppelelemente (14) jeweils mit demjenigen ersten Koppelelement (13) koppeln, in dessen erstem Winkelbereich sich der zweite Winkelbereich des jeweiligen zweiten Koppelelements (14) gerade befindet, und
- wobei ein Schaltzustand einer zwischen den zweiten Koppelelementen (14) und den zweiten Modems (16) angeordneten Schaltmatrix (15) von einer Einstelleinrichtung (18) dynamisch derart eingestellt wird, dass die ersten Daten stets zwischen denselben ersten und zweiten Modems (12, 16) übertragen werden,
**dadurch gekennzeichnet,**
**dass** mittels zwischen den ersten Modems (12) und den ersten Koppelelementen (13) und/oder zwischen den zweiten Modems (16) und den zweiten Koppelelementen (14) angeordneter Verzerrungseinrichtungen (23, 24) eine Verzerrung des jeweils übertragenen Signals gemäß einer jeweiligen Verzerrungsvorschrift erfolgt und dass die jeweilige Verzerrungsvorschrift von der Einstelleinrichtung (18) in Abhängigkeit vom Drehwinkel (φ) des ersten Teils (3) relativ zum zweiten Teil (2) eingestellt wird.

2. Betriebsverfahren nach Anspruch 1,
**dadurch gekennzeichnet,**
**dass** die Einstelleinrichtung (18) den Schaltzustand der Schaltmatrix (15) in Abhängigkeit von einem Drehwinkel (φ) des ersten Teils (3) relativ zum zweiten Teil (2) ermittelt.

3. Betriebsverfahren nach Anspruch 2,
**dadurch gekennzeichnet,**
**dass** die Einstelleinrichtung (18) im Rahmen der Ermittlung des Schaltzustands der Schaltmatrix (15) zusätzlich zu dem jeweiligen Drehwinkel (φ) mindestens eine zeitliche Ableitung des Drehwinkels (φ) berücksichtigt.

4. Betriebsverfahren nach Anspruch 1, 2 oder 3,
**dadurch gekennzeichnet,**
**dass** die zu übertragenden Daten den ersten oder zweiten Modems (12, 16) als komplexe Signale zugeführt werden und dass die ersten oder zweiten Modems (12, 16) eine Quadraturamplitudenmodulation eines Trägersignals (TS) gemäß den ihnen zugeführten komplexen Signalen durchführen.

5. Betriebsverfahren nach einem der obigen Ansprüche,
**dadurch gekennzeichnet,**
**dass** während der Rotation des ersten Teils (3) relativ zum zweiten Teil (2) über die ersten und zweiten Koppelelemente (13, 14) zusätzlich zwischen auf dem ersten Teil (3) angeordneten dritten Modems (25) und auf dem zweiten Teil (2) angeordneten vierten Modems (26) zweite Daten übertragen werden und dass die Schaltmatrix (15) von der Einstelleinrichtung (18) dynamisch derart geschaltet wird, dass die zweiten Daten stets zwischen denselben dritten und vierten Modems (25, 26) übertragen werden.

6. Betriebsverfahren nach Anspruch 5,
**dadurch gekennzeichnet,**
**dass** die ersten und die zweiten Daten mittels der ersten bis vierten Modems (12, 16, 25, 26) vor dem Zuführen zu den ersten und zweiten Koppelelementen (13, 14) auf Trägersignale verschiedener Frequenzen (f) aufmoduliert werden, so dass die jeweils belegten Frequenzbereiche zueinander disjunkt sind.

7. Computertomograph,
- wobei der Computertomograph ein erstes und ein zweites Teil (3, 2) aufweist,
- wobei das erste Teil (3) relativ zum zweiten Teil (2) um eine Rotationsachse (4) rotierbar ist,
- wobei auf dem ersten Teil (3) eine Mehrzahl von ersten Modems (12) angeordnet ist, die mit auf dem ersten Teil (3) angeordneten ersten Koppelelementen (13) datentechnisch verbunden sind, und auf dem zweiten Teil (2) eine Mehrzahl von zweiten Modems (16) angeordnet ist, die mit auf dem zweiten Teil (2) angeordneten zweiten Koppelelementen (14) datentechnisch verbunden sind,
- wobei die ersten Koppelelemente (13) sich in Umfangsrichtung um die Rotationsachse (4) herum gesehen über einen jeweiligen ersten Winkelbereich erstrecken,
- wobei die ersten Winkelbereiche von erstem Koppelelement (13) zu erstem Koppelelement (13) gesehen zueinander disjunkt sind und über die Gesamtheit der ersten Koppelelemente (13) gesehen einen Vollkreis um die Rotationsachse (4) herum bilden,
- wobei die zweiten Koppelelemente (14) sich um die Rotationsachse (4) herum gesehen über einen jeweiligen zweiten Winkelbereich erstrecken,
- wobei die zweiten Winkelbereiche um die Rotationsachse (4) herum gesehen zueinander disjunkt und voneinander beabstandet sind, insbesondere gleichmäßig um die Rotationsachse (4) herum verteilt angeordnet sind,
- so dass die zweiten Koppelelemente (14) jeweils mit demjenigen ersten Koppelelement (13) koppeln, in dessen erstem Winkelbereich sich der zweite Winkelbereich des jeweiligen zweiten Koppelelements (14) gerade befindet,
- wobei zwischen den zweiten Koppelelementen (14) und den zweiten Modems (16) eine Schaltmatrix (15) angeordnet ist und
- wobei der Schaltmatrix (15) eine Einstelleinrichtung (18) zugeordnet ist, von der ein Schaltzustand der Schaltmatrix (15) dynamisch derart eingestellt wird, dass während einer während der Rotation des ersten Teils (3) relativ zum zweiten Teil (2) um die Rotationsachse (4) erfolgenden Datenübertragung erster Daten zwischen den ersten und den zweiten Modems (12, 16) die ersten Daten stets zwischen denselben ersten und zweiten Modems (12, 16) übertragen werden,
**dadurch gekennzeichnet,**
**dass** zwischen den ersten Modems (12) und den ersten Koppelelementen (13) und/oder zwischen den zweiten Modems (16) und den zweiten Koppelelementen (14) Verzerrungseinrichtungen (23, 24) angeordnet sind, mittels derer eine Verzerrung des jeweils übertragenen Signals gemäß einer jeweiligen Verzerrungsvorschrift erfolgt, und dass die jeweilige Verzerrungsvorschrift von der Einstelleinrichtung (18) in Abhängigkeit vom Drehwinkel (φ) des ersten Teils (3) relativ zum zweiten Teil (2) eingestellt wird.

8. Computertomograph nach Anspruch 7,
**dadurch gekennzeichnet,**
**dass** die Einstelleinrichtung (18) den Schaltzustand der Schaltmatrix (15) in Abhängigkeit von einem Drehwinkel (φ) des ersten Teils (3) relativ zum zweiten Teil (2) ermittelt.

9. Computertomograph nach Anspruch 8,
**dadurch gekennzeichnet,**
**dass** die Einstelleinrichtung (18) im Rahmen der Ermittlung des Schaltzustands der Schaltmatrix (15) zusätzlich zu dem jeweiligen Drehwinkel (φ) mindestens eine zeitliche Ableitung des Drehwinkels (φ) berücksichtigt.

10. Computertomograph nach Anspruch 7, 8 oder 9,
**dadurch gekennzeichnet,**
**dass** die zu übertragenden Daten den ersten oder zweiten Modems (12, 16) als komplexe Signale zugeführt werden und dass die ersten oder zweiten Modems (12, 16) als Quadraturamplitudenmodulatoren ausgebildet sind, die eine Quadraturamplitudenmodulation eines Trägersignals (TS) gemäß den ihnen zugeführten komplexen Signalen durchführen.

11. Computertomograph nach einem der Ansprüche 7 bis 10,
**dadurch gekennzeichnet,**
**dass** auf dem ersten Teil (3) eine Mehrzahl von dritten Modems (25) angeordnet ist, die mit den ersten Koppelelementen (13) datentechnisch verbunden sind, und auf dem zweiten Teil (2) eine Mehrzahl von vierten Modems (26) angeordnet ist, die mit den zweiten Koppelelementen (14) über die Schaltmatrix (15) datentechnisch verbunden sind, so dass während der Rotation des ersten Teils (3) relativ zum zweiten Teil (2) über die ersten und zweiten Koppelelemente (13, 14) zusätzlich zwischen den dritten Modems (25) und den vierten Modems (26) zweite Daten übertragen werden, und dass die Schaltmatrix (15) von der Einstelleinrichtung (18) dynamisch derart geschaltet wird, dass während einer während der Rotation des ersten Teils (3) relativ zum zweiten Teil (2) erfolgenden Datenübertragung der zweiten Daten die zweiten Daten stets zwischen denselben dritten und vierten Modems (25, 26) übertragen werden.

12. Computertomograph nach Anspruch 11,
**dadurch gekennzeichnet,**
**dass** die ersten und die zweiten Daten mittels der ersten bis vierten Modems (12, 16, 25, 26) vor dem Zuführen zu den ersten und zweiten Koppelelementen (13, 14) auf Trägersignale verschiedener Frequenzen (f) aufmoduliert werden, so dass die jeweils belegten Frequenzbereiche zueinander disjunkt sind.

## Claims

1. Operating method for a computed tomography system (1),
- wherein during a rotation of a first part (3) of the computed tomography system (1) relative to a second part (2) of the computed tomography system (1) around an axis of rotation (4) between a plurality of first modems (12) arranged on the first part (3) and a plurality of second modems (16) arranged on the second part (2), first data is transmitted via a plurality of first coupling elements (13) arranged on the first part (3) and a plurality of second coupling elements (14) arranged on the second part (2),
- wherein the first coupling elements (13) extend over a respective first angular range when viewed in the circumferential direction around the axis of rotation (4),
- wherein the first angular ranges, viewed from the first coupling element (13) to the first coupling element (13), are disjoint in relation to one another and, viewed over the entirety of the first coupling elements (13), form a full circle around the axis of rotation (4),
- wherein the second coupling elements (14) extend over a respective second angular range as viewed around the axis of rotation (4),
- wherein the second angular ranges, as viewed around the axis of rotation (4), are disjoint in relation to one another and spaced apart from one another, in particular evenly distributed around the axis of rotation (4),
- so that the second coupling elements (14) each couple with the first coupling element (13) in whose first angular range the second angular range of the respective second coupling element (14) is currently located, and
- wherein a switching state of a switching matrix (15) arranged between the second coupling elements (14) and the second modems (16) is dynamically set by a setting facility (18) in such a way that the first data is always transmitted between the same first and second modems (12, 16),
**characterised in that**
by means of distortion facilities (23, 24) arranged between the first modems (12) and the first coupling elements (13) and/or between the second modems (16) and the second coupling elements (14), the respectively transmitted signal is distorted in accordance with a respective distortion rule, and **in that** the respective distortion rule is set by the setting facility (18) as a function of the angle of rotation (φ) of the first part (3) relative to the second part (2).

2. Operating method according to claim 1,
**characterised in that**
the setting facility (18) determines the switching state of the switching matrix (15) as a function of an angle of rotation (φ) of the first part (3) relative to the second part (2).

3. Operating method according to claim 2,
**characterised in that**
the setting facility (18) takes into account at least one time derivative of the angle of rotation (φ) in addition to the respective angle of rotation (φ) when determining the switching state of the switching matrix (15).

4. Operating method according to claim 1, 2 or 3,
**characterised in that**
the data to be transmitted is supplied to the first or second modems (12, 16) as complex signals, and **in that** the first or second modems (12, 16) perform quadrature amplitude modulation of a carrier signal (TS) in accordance with the complex signals supplied to them.

5. Operating method according to one of the above claims,
**characterised in that**
during the rotation of the first part (3) relative to the second part (2), second data is additionally transmitted via the first and second coupling elements (13, 14) between third modems (25) arranged on the first part (3) and fourth modems (26) arranged on the second part (2), and **in that** the switching matrix (15) is dynamically switched by the setting facility (18) in such a way that the second data is always transmitted between the same third and fourth modems (25, 26).

6. Operating method according to claim 5,
**characterised in that**
the first and the second data is modulated onto carrier signals of different frequencies (f) by means of the first to fourth modems (12, 16, 25, 26) before being fed to the first and second coupling elements (13, 14), so that the frequency ranges occupied in each case are disjoint in relation to one another.

7. Computed tomography system,
- wherein the computed tomography system comprises a first and a second part (3, 2),
- wherein the first part (3) is rotatable relative to the second part (2) around an axis of rotation (4),
- wherein a plurality of first modems (12) is arranged on the first part (3) which are connected in a data-technical manner to first coupling elements (13) arranged on the first part (3), and a plurality of second modems (16) is arranged on the second part (2) which are connected in a data-technical manner to second coupling elements (14) arranged on the second part (2),
- wherein the first coupling elements (13) extend over a respective first angular range when viewed in the circumferential direction around the axis of rotation (4),
- wherein the first angular ranges, viewed from the first coupling element (13) to the first coupling element (13), are disjoint in relation to one another and, viewed over the entirety of the first coupling elements (13), form a full circle around the axis of rotation (4),
- wherein the second coupling elements (14) extend over a respective second angular range as viewed around the axis of rotation (4),
- wherein the second angular ranges, as viewed around the axis of rotation (4), are disjoint in relation to one another and spaced apart from one another, in particular evenly distributed around the axis of rotation (4),
- so that the second coupling elements (14) each couple with the first coupling element (13) in whose first angular range the second angular range of the respective second coupling element (14) is currently located,
- wherein a switching matrix (15) is arranged between the second coupling elements (14) and the second modems (16) and
- wherein the switching matrix (15) is assigned a setting facility (18), by which a switching state of the switching matrix (15) is dynamically set in such a way that during data transmission of first data between the first and the second modems (12, 16), which takes place during the rotation of the first part (3) relative to the second part (2) around the axis of rotation (4), the first data is always transmitted between the same first and second modems (12, 16),
**characterised in that**
distortion facilities (23, 24) are arranged between the first modems (12) and the first coupling elements (13) and/or between the second modems (16) and the second coupling elements (14), by means of which the respectively transmitted signal is distorted in accordance with a respective distortion rule, and **in that** the respective distortion rule is set by the setting facility (18) as a function of the angle of rotation (φ) of the first part (3) relative to the second part (2).

8. Computed tomography system according to claim 7,
**characterised in that**
the setting facility (18) determines the switching state of the switching matrix (15) as a function of an angle of rotation (φ) of the first part (3) relative to the second part (2).

9. Computed tomography system according to claim 8,
**characterised in that**
the setting facility (18) takes into account at least one time derivative of the angle of rotation (φ) in addition to the respective angle of rotation (φ) when determining the switching state of the switching matrix (15).

10. Computed tomography system according to claim 7, 8 or 9,
**characterised in that**
the data to be transmitted is supplied to the first or second modems (12, 16) as complex signals, and **in that** the first or second modems (12, 16) are designed as quadrature amplitude modulators which perform quadrature amplitude modulation of a carrier signal (TS) in accordance with the complex signals supplied to them.

11. Computed tomography system according to one of claims 7 to 10,
**characterised in that**
a plurality of third modems (25) is arranged on the first part (3), which is connected to the first coupling elements (13) in a data-technical manner, and a plurality of fourth modems (26) is arranged on the second part (2), which is connected to the second coupling elements (14) in a data-technical manner via the switching matrix (15), so that during the rotation of the first part (3) relative to the second part (2), second data is additionally transmitted between the third modems (25) and the fourth modems (26) via the first and second coupling elements (13, 14), and **in that** the switching matrix (15) is dynamically switched by the setting facility (18) in such a way that, during data transmission of the second data which takes place during the rotation of the first part (3) relative to the second part (2), the second data is always transmitted between the same third and fourth modems (25, 26).

12. Computed tomography system according to claim 11,
**characterised in that**
the first and the second data is modulated onto carrier signals of different frequencies (f) by means of the first to fourth modems (12, 16, 25, 26) before being fed to the first and second coupling elements (13, 14), so that the frequency ranges occupied in each case are disjoint in relation to one another.

## Revendications

1. Procédé pour faire fonctionner un tomodensitomètre (1) assisté par ordinateur,
- dans lequel pendant une rotation d'une première partie (3) du tomodensitomètre (1) assisté par ordinateur par rapport à une deuxième partie (2) du tomodensitomètre (1) assisté par ordinateur autour d'un axe (4) de rotation, on transmet des premières données entre une pluralité de premiers modems (12) disposés sur la première partie (3) et une pluralité de deuxièmes modems (16) disposés sur la deuxième partie (2) en passant par une pluralité de premiers éléments (13) de raccordement montés sur la première partie (3) et d'une pluralité de deuxièmes éléments (14) de raccordement montés sur une deuxième partie (2),
- dans lequel les premiers éléments (13) de raccordement s'étendent sur une première plage angulaire respective, considéré dans la direction périphérique autour de l'axe (4) de rotation,
- dans lequel les premières plages angulaires du premier élément (13) de raccordement, sont, considéré par rapport au premier élément (13) de raccordement, disjointes l'une de l'autre et forment, considéré sur la totalité des premiers éléments (13) de raccordement, un cercle complet autour de l'axe (4) de rotation,
- dans lequel les deuxièmes éléments (14) de raccordement s'étendent sur une deuxième plage angulaire respective, considéré autour de l'axe (4) de rotation,
- dans lequel les deuxièmes plages angulaires sont, considéré autour de l'axe (4) de rotation, disjointes les unes des autres et à distance les unes des autres, en étant en particulier, réparties uniformément autour de l'axe (4) de rotation,
- de sorte que les deuxièmes éléments (14) de raccordement se raccordent respectivement au premier élément (13) de raccordement, dans la première plage angulaire duquel se trouve précisément la deuxième plage angulaire de l'élément (14) de raccordement respectif, et
- dans lequel on règle dynamiquement, par un dispositif (18) de réglage, un état de commutation d'une matrice (15) de commutation disposée entre les deuxièmes éléments (14) de raccordement et les deuxièmes modems (16), de manière à transmettre les premières données toujours entre les mêmes premier et deuxième modems (12, 16), **caractérisé**
**en ce qu'**au moyen de dispositifs (23, 24) de distorsion disposés entre les premiers modems (12) et les premiers éléments (13) de raccordement et/ou entre les deuxièmes modems (16) et les deuxièmes éléments (14) de raccordement, il se produit une distorsion du signal respectif transmis suivant une règle de distorsion respective et en ce que la règle de distorsion respective est réglée par le dispositif (18) de réglage en fonction de l'angle (φ) de rotation de la première partie (3) par rapport à la deuxième partie (2).

2. Procédé suivant la revendication 1,
**caractérisé**
**en ce que** le dispositif (18) de réglage détermine l'état de commutation de la matrice (15) de commutation en fonction d'un angle (φ) de rotation de la première partie (3) par rapport à la deuxième partie (2).

3. Procédé suivant la revendication 2,
**caractérisé**
**en ce que** le dispositif (18) de réglage tient compte, dans le cadre de la détermination de l'état de commutation de la matrice (15) de commutation, supplémentairement à l'angle (φ) respectif de rotation, au moins d'une dérivée en fonction du temps de l'angle (φ) de rotation.

4. Procédé suivant la revendication 1, 2 ou 3,
**caractérisé**
**en ce que** l'on envoie, comme signaux complexes, les données à transmettre aux premiers ou aux deuxièmes modems (12, 16) et en ce que les premiers ou les deuxièmes modems (12, 16) effectuent une modulation d'amplitude en quadrature d'un signal (TS) porteur suivant les signaux complexes, qui leur sont envoyés.

5. Procédé suivant l'une des revendications précédentes,
**caractérisé**
**en ce que** pendant la rotation de la première partie (3) par rapport à la deuxième partie (2), on transmet des deuxièmes données en passant par les premiers et les deuxièmes éléments (13, 14) de raccordement supplémentairement entre des troisièmes modems (25) disposés sur la première partie (3) et des quatrièmes modems (26) disposés sur la deuxième partie (2), et en ce que l'on commute dynamiquement la matrice (15) de commutation par le dispositif (18) de réglage, de manière à transmettre les deuxièmes données toujours entre les mêmes troisième et quatrième modems (25, 26).

6. Procédé suivant la revendication 5,
**caractérisé**
**en ce que** l'on module, sur des signaux porteurs de fréquences (f) différentes, les premières et les deuxièmes données au moyen des premiers à quatrièmes modems (12, 16, 25, 26) avant l'envoi aux premier et deuxième éléments (13, 14) de raccordement, de manière à ce que les domaines de fréquence occupés respectivement soient disjoints les uns des autres.

7. Tomodensitomètre assisté par ordinateur,
- dans lequel le tomodensitomètre assisté par ordinateur a une première et une deuxième parties (3, 2),
- dans lequel la première partie (3) peut tourner par rapport à la deuxième partie (2) autour d'un axe (4) de rotation,
- dans lequel sur la première partie (3), est disposée une pluralité de premiers modems (12), qui sont raccordés en technique des données à des premiers éléments (13) de raccordement disposés sur la première partie (3), et sur la deuxième partie (2) est disposée une pluralité de deuxièmes modems (16), qui sont raccordés en technique des données à des deuxièmes éléments (14) de raccordement disposés sur la deuxième partie (2),
- dans lequel les premiers éléments (13) de raccordement s'étendent sur une première plage angulaire respective, considéré dans la direction périphérique autour de l'axe (4) de rotation,
- dans lequel les premières plages angulaires du premier élément (13) de raccordement, sont, considéré par rapport au premier élément (13) de raccordement, disjointes l'une de l'autre et forment, considéré sur la totalité des premiers éléments (13) de raccordement, un cercle complet autour de l'axe (4) de rotation,
- dans lequel les deuxièmes éléments (14) de raccordement s'étendent, considéré sur une deuxième plage angulaire respective, autour de l'axe (4) de rotation,
- dans lequel les deuxièmes plages angulaires sont, considéré autour de l'axe (4) de rotation, disjointes les unes des autres et à distance les unes des autres, en étant, en particulier, réparties uniformément autour de l'axe (4) de rotation,
- de sorte que les deuxièmes éléments (14) de raccordement se raccordent respectivement au premier élément (13) de raccordement, dans la première plage angulaire duquel se trouve précisément la deuxième plage angulaire du deuxième élément (14) de raccordement respectif,
- dans lequel une matrice (15) de commutation est disposée entre les deuxièmes éléments (14) de raccordement et les deuxièmes modems (16), et
- dans lequel, à la matrice (15) de commutation est affecté un dispositif (18) de réglage, par lequel un état de commutation de la matrice (15) de commutation est réglé dynamiquement, de manière à ce que, pendant une transmission de données, s'effectuant pendant la rotation de la première partie (3) par rapport à la deuxième partie (2) autour de l'axe (4) de rotation, de premières données entre les premiers et les deuxièmes modems (12, 16), les premières données soient transmises toujours entre les mêmes premier et deuxième modems (12, 16),
**caractérisé**
**en ce que**, entre les premiers modems (12) et les premiers éléments (13) de raccordement et/ou entre les deuxièmes modems (16) et les deuxièmes éléments (14) de raccordement, sont disposés des dispositifs (23, 24) de distorsion, au moyen desquels une distorsion des signaux transmis respectivement a lieu suivant une règle de distorsion respective, et en ce que la règle de distorsion respective est réglée par le dispositif (18) de réglage en fonction de l'angle (φ) de rotation de la première partie (3) par rapport à la deuxième partie (2).

8. Tomodensitomètre assisté par ordinateur suivant la revendication 7,
**caractérisé**
**en ce que** le dispositif (18) de réglage détermine l'état de commutation de la matrice (15) de commutation en fonction d'un angle (φ) de rotation de la première partie (3) par rapport à la deuxième partie (2).

9. Tomodensitomètre assisté par ordinateur suivant la revendication 8,
**caractérisé**
**en ce que** le dispositif (18) de réglage tient compte, dans le cadre de la détermination de l'état de commutation de la matrice (15) de commutation, supplémentairement à l'angle (φ) respectif de rotation, au moins d'une dérivée en fonction du temps de l'angle (φ) de rotation.

10. Tomodensitomètre assisté par ordinateur suivant la revendication 7, 8 ou 9,
**caractérisé**
**en ce que** l'on envoie, comme signaux complexes, les données à transmettre aux premiers ou aux deuxièmes modems (12, 16) et en ce que les premiers ou les deuxièmes modems (12, 16) effectuent une modulation d'amplitude en quadrature d'un signal (TS) porteur suivant les signaux complexes, qui leur sont envoyés.

11. Tomodensitomètre assisté par ordinateur suivant l'une des revendications 7 à 10,
**caractérisé**
**en ce que** sur la première partie (3), est disposée une pluralité de troisièmes modems (25), qui sont en technique des données raccordés aux premiers éléments (13) de raccordement et sur la deuxième partie (2), est disposée une pluralité de quatrièmes modems (26), qui sont raccordés, en technique des données, aux deuxièmes éléments (14) de raccordement par l'intermédiaire de la matrice (15) de commutation, de manière à ce que, pendant la rotation de la première partie (3) par rapport à la deuxième partie (2), soient transmises, en passant par les premiers et deuxièmes éléments (13, 14) supplémentairement des deuxièmes données entre les troisièmes modems (25) et les quatrièmes modems (26), et en ce que la matrice (15) de commutation est commutée dynamiquement par le dispositif (18) de réglage, de manière à transmettre, pendant une transmission des deuxièmes données, s'effectuant par la rotation de la première partie (3) par rapport à la deuxième partie (2), les deuxièmes données toujours entre les mêmes troisième et quatrième modems (25, 26).

12. Tomodensitomètre assisté par ordinateur suivant la revendication 11,
**caractérisé**
**en ce que** l'on module sur des signaux porteurs de fréquences (f) différentes, les premières et les deuxièmes données au moyen des premiers à quatrièmes modems (12, 16, 25, 26) avant l'envoi aux premiers et deuxièmes éléments (13, 14) de raccordement, de manière à ce que les domaines de fréquence occupés respectivement soient disjoints les uns des autres.
